# EUROPEAN PATENT SPECIFICATION

(11) **EP 4 295 764 B1**
(45) Date of publication and mention of the grant of the patent: **18.11.2026**
(21) Application number: 21854854.3
(22) Date of filing: 19.02.2021
(51) Int. Cl.: A61B 5/00

(54) **SPINAL ALIGNMENT-ESTIMATING APPARATUS, SYSTEM FOR ESTIMATING SPINAL ALIGNMENT, METHOD FOR ESTIMATING SPINAL ALIGNMENT, PROGRAM FOR ESTIMATING SPINAL ALIGNMENT, AND NON-TRANSITORY COMPUTER-READABLE RECORDING MEDIUM HAVING STORED THEREIN PROGRAM FOR ESTIMATING SPINAL ALIGNMENT**
WIRBELSÄULENAUSRICHTUNGSSCHÄTZVORRICHTUNG, SYSTEM ZUM SCHÄTZEN EINER WIRBELSÄULENAUSRICHTUNG, VERFAHREN ZUM SCHÄTZEN EINER WIRBELSÄULENAUSRICHTUNG, PROGRAMM ZUM SCHÄTZEN EINER WIRBELSÄULENAUSRICHTUNG UND NICHTTRANSITORISCHES COMPUTERLESBARES AUFZEICHNUNGSMEDIUM, AUF DEM EIN PROGRAMM ZUM SCHÄTZEN EINER WIRBELSÄULENAUSRICHTUNG GESPEICHERT IST
DISPOSITIF D'ESTIMATION D'ALIGNEMENT VERTÉBRAL, SYSTÈME D'ESTIMATION D'ALIGNEMENT VERTÉBRAL, PROCÉDÉ D'ESTIMATION D'ALIGNEMENT VERTÉBRAL, PROGRAMME D'ESTIMATION D'ALIGNEMENT VERTÉBRAL ET SUPPORT D'ENREGISTREMENT NON TRANSITOIRE LISIBLE PAR ORDINATEUR SUR LEQUEL EST STOCKÉ UN PROGRAMME D'ESTIMATION D'ALIGNEMENT VERTÉBRAL

(43) Date of publication of application: 27.12.2023
(73) Proprietor: Tohoku University, Sendai-shi, Miyagi 980-8577 (JP)
(72) Inventor: TAKAHASHI, Yusei, Sendai-shi, Miyagi 980-8577 (JP); HASHIMOTO, Ko, Sendai-shi, Miyagi 980-8577 (JP)
(74) Representative: Hoffmann Eitle
(86) International application number: PCT/JP2021/006490
(87) International publication number: WO 2022/176176

(56) References cited:
- WO-A1-2016/079585
- WO-A1-2017/141958
- JP-A- 2014 090 841
- JP-A- 2015 107 141
- JP-A- 2020 054 433
- JP-A- 2020 074 876
- US-A1- 2019 038 215
- US-A1- 2019 313 967
- LEE HOSUB ET AL: "Mobile posture monitoring system to prevent physical health risk of smartphone users", LOW POWER ELECTRONICS AND DESIGN, ACM, 2 PENN PLAZA, SUITE 701 NEW YORK NY 10121-0701 USA, 5 September 2012 (2012-09-05), pages 592 - 593, XP059221647, ISBN: 978-1-4503-4185-1, DOI: 10.1145/2370216.2370320
- HAN HOBEOM ET AL: "Novel Wearable Monitoring System of Forward Head Posture Assisted by Magnet-Magnetometer Pair and Machine Learning", IEEE SENSORS JOURNAL, IEEE, USA, vol. 20, no. 7, 16 December 2019 (2019-12-16), pages 3838 - 3848, XP011776944, ISSN: 1530-437X, [retrieved on 20200305], DOI: 10.1109/JSEN.2019.2959817
- KATO YOSHINA, FUKUDA SAKI, SUZUKI YASUO, OTA SUSUMU: " Effect of Posture Feedback Band on Posture Alignment and Pain during a Visual Display Terminal Task", VDT (VISUAL DISPLAY TERMINALS), vol. 16, no. 4, 1 December 2016 (2016-12-01), pages 315 - 319, XP055964938

## Description

### [TECHNICAL FIELD]

The present invention relates to a spinal alignment-estimating apparatus, a system for estimating a spinal alignment, a method for estimating a spinal alignment, and a program for estimating a spinal alignment.

### [BACKGROUND ART]

With the widespread use of Visual Display Terminals (VDT) such as computer displays, problems of mental and physical symptoms (VDT syndromes) caused by long VDT operations have been regarded have arisen.

In particular, when using a portable VDT such as a laptop computer or a smart phone, users tend to lean forward. Persistent forward-leaning posture places a load on the muscles surrounding the cervical spine that supports the weight of the head and causes various symptoms such as stiff neck and headache.

A wearable device that can be put or worn on the body and a terminal device that can be incorporated into the VDT itself when being used have been known as examples of a device that monitors the position in the anteroposterior direction of the head-and-neck of the subject using the VDT over time and determines whether the user is in a bad posture and alert the subject.

As an example of the wearable-type device, a lensless frame having an inclination sensor measures the slope angle in the front-rear direction of the frame with respect to the horizontal axis (viewing angle 0 °), and alerts the user when the slope angle is outside a predetermined tolerance range (see Patent Document 1).

As another example of the wearable-type device, smart eyeglasses provided with multiple sensors alerts the user when the slope angle and the pitch angle in the anteroposterior direction of the head of the user measured by an inertial sensor exceed respective predetermined tolerance values, or the distance to the target measured by a distance sensor is less than the set value for a given time period or longer (see Patent Document 2).

On the other hand, as an example of the terminal-type device, a mobile terminal incorporating therein multiple sensors calculates the angle in the front-rear direction in the space of the mobile terminal with an acceleration sensor, measures the distance between the screen and the user's eyes with a distance sensor, determines the user's posture from these two pieces of information, and alerts the user (see Patent Document 3).

### [Prior art documents]

### [Patent Document]

[Patent Document 1] WO 2016/079585
[Patent Document 2] Chinese Patent Publication No. 103479361
[Patent Document 3] Japanese Laid-open Patent publication No. 2015-39472
[Patent Document 4] WO 2016/079 585 A1

### [Non Patent Document]

Lee Hosub et al.: "Mobile Posture Monitoring System to Prevent Physical Health Risk of Smartphone Users" Low Power Electronics and Design
WO 2016/079 585 A1 describes a wearable posture monitoring and feedback system and a related method configured to monitor and provide feedback regarding a user's posture while operating and viewing a portable electronic device including: a wearable frame configured to be fit on or about a head of the user; a sensor mounted on the wearable frame configured to monitor at least a head position of the user; and a transmitter configured to transmit data related to at least the head position wirelessly from the sensor to a receiver on or in the portable electronic device. The system is configured to (1 ) provide feedback to the user based on the data from the sensor; and (2) send a signal to alter an output provided to the user by the portable electronic device based on the data from the sensor.

### [SUMMARY OF THE INVENTION]

### [PROBLEM TO BE SOLVED BY THE INVENTION]

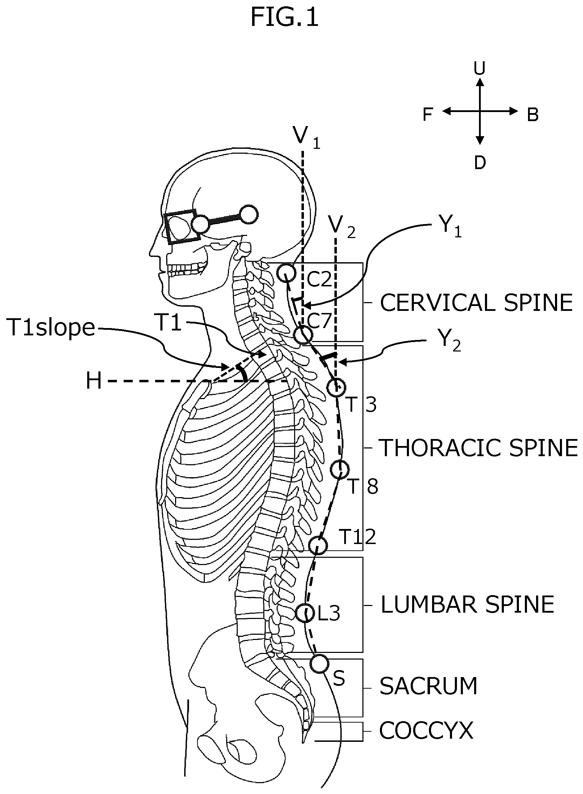

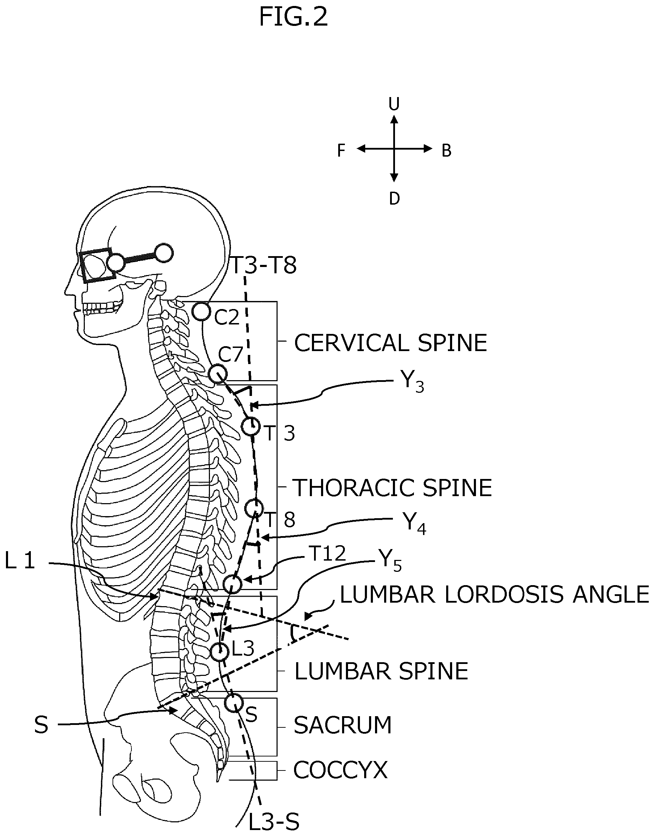

In each of the above methods of determining a posture focuses only on the head-and-neck of a subject, and determines the posture based on whether or not the slope angle of the head-and-neck in the anteroposterior direction and the distance between the head-and-neck and VDT are within the tolerance ranges. However, a postural change, particularly in the sitting posture, involves not only the head-and-neck, but also all of positions of the cervical spine, the thoracic spine, the lumbar spine, and the pelvis that constituting the spine (see FIGs. 1 and 2). Therefore, there is a possibility that the posture of the subject is not accurately estimated by the posture determination based only on the information on the head-and-neck.

Usually, information of arrangement of spine (spinal alignment) of a human is obtained from a three-dimensional motion analyzer such as a motion capture or from X-ray imaging. However, the motion analyzer requires an expensive large-scale apparatus, and therefore has difficulty to use on a daily basis for everyone. X-ray imaging involves radiation exposure and is usually not acceptable unless there are justifiable medical reasons. In addition, it is impossible to dynamically obtain the posture of the subject using a VDT over time.

In one aspect, the object of the techniques described herein are directed to estimate a spinal alignment of a subject person in a sitting posture over time, dynamically, and accurately with a simple device.

### [MEANS TO SOLVE THE PROBLEM]

The above problem is solved by the subject matter of the independent claims. Examples and technical descriptions of apparatuses, products and/or methods in the description and/or drawings which are not covered by the claims are presented not as embodiments of the invention but as background art or examples useful for understanding the invention.

### [EFFECT OF THE INVENTION]

In one aspect, the spinal alignment of a subject person in a sitting posture can be estimated over time, dynamically, and accurately with a simple device.

### [BRIEF DESCRIPTION OF DRAWINGS]

FIG. 1 is a diagram illustrating an index used for estimating a spinal alignment of a user;
FIG. 2 is a diagram illustrating an index used for estimating a spinal alignment of a user;
FIG. 3 is a diagram illustrating a system for estimating a spinal alignment according to an embodiment;
FIG. 4 is a diagram illustrating an appearance structure of a measuring terminal of one embodiment attached to eyeglasses;
FIG. 5 is a diagram illustrating a distance between the head of a user and a VDT and an angle of the head of the user;
FIG. 6A and FIG.6B are diagrams illustrating fluctuation of the spinal alignment of the user in a sitting posture;
FIG. 7 is a diagram illustrating a correlation between a cervical spine slope angle and a viewing distance;
FIG. 8 is a diagram illustrating a correlation between the cervical spine slope angle and a head slope angle;
FIG. 9 is a diagram showing an example of a result of multiple regression analysis using coefficients being related to alignment of each part of the spine and being obtained by using data obtained by an apparatus and a result of measuring with a three-dimensional motion analyzer;
FIG. 10 is a flow diagram illustrating an example of operating of the system for estimating a spinal alignment of the one embodiment; and
FIG. 11 is a block diagram illustrating an example of a hardware configuration of a computer of the one embodiment.

### [EMBODIMENTS TO CARRY OUT INVENTION]

Hereinafter, an exemplary embodiment will now be described with reference to the accompanying drawings, it is understood that the invention is defined by the appended claims.

In the drawings to be used in the following description, like reference numbers denote the same or similar parts, unless otherwise specified.

In the embodiment, the directions used in the description are defined as follows. The horizontal direction will be described in detail in the front-rear direction (the front is indicated by "F" and the rear is indicated by "B" in the drawings) and in the left-right direction (the left is indicated by "L" and the right is indicated by "R" in the drawings). The left and right directions are determined on the basis of a state directing from the rear to the front. In addition, the direction that the gravity acts among the vertical direction is downward (indicated by "D" in the drawings) and the opposite direction of the downward is defined as upward (indicated by "U" in the drawings).

### [1] One embodiment

### [1-1] Definition of the human spine

Here, the terms related to the human spine and the reference symbols for illustrative purposes of the present application will now be defined.

FIGs. 1 and 2 are diagrams each illustrating an index used for estimating a spinal alignment of a human.
The human spine is composed of the cervical spine, the thoracic spine, the lumbar spine, the sacral, and the coccyx, and the arrangement of these bones is referred to as the spinal alignment. Bones are abbreviated from English-language acronyms, i.e., the cervical spine is represented by C1-C7; the thoracic spine is represented by T1-T12; the lumbar spine is represented by L1-L5; the sacrum is represented by S; and the coccyx is represented by Co. In the technique described in this specification, for estimating the posture of the user operating a VDT in the sitting posture uses the body surface median at the heights corresponding to the second cervical spine C2, the seventh cervical spine C7, the third thoracic spine T3, the eighth thoracic spine T8, the twelfth thoracic spine T12, the third lumbar spine L3, and the sacrum S is used as the reference points. The white circles (markers) arranged in FIGS. 1 and 2 indicate the position of the body surface corresponding to the positions of the bones related to the posture of the user in the sitting posture.

As described above, connecting the centers of the white circles arranged on the body surface corresponding to the median of C2, C7, T3, T8, T12, L3, and S, which are the reference points for estimating the posture of the user in the sitting posture, by a straight line obtains one polygonal line drawn along the back surface of the user. The polygonal line is a line that roughly indicates the spinal alignment in the anteroposterior direction of the user. The angles that lines connecting points form in conjunction with the horizontal line and vertical line and the angles formed by the two adjacent lines approximate the degrees of the slopes and curvature of the respective parts of the spine.

The angles in this section are angles in the anteroposterior (sagittal) direction of the spinal column. In FIG. 1, the angle (C2-C7 vertical angle) formed by the line segment connecting the C2 and the C7 and the vertical line V₁ is represented by Y₁. The angle Y₁ is the angle representing the degree of leaning forward of the cervical spine. In FIG. 1, the angle formed by a line segment connecting the C7 and the T3 and the vertical line V₂ is represented by Y₂. The angle Y₂ approximates the T1 slope that is a key indicator to determine the cervical spinal alignment, which corresponds to the angle that the upper surface of the first thoracic vertebral body (T1) forms in conjunction with the horizontal line H shown in FIG. 1. The T1 slope indicates how many degrees the upper surface of the vertebral body of the first thoracic spine is inclined with respect to the horizontal plane, and is clinically one of the indices that defines the cervical spinal alignment.

In FIG. 2, a sharp angle formed by a line segment connecting the C7 and the T3 and a line segment obtained by extending upward a line segment connecting the T3 and the T8 is represented by Y₃. The angle Y₃ approximates the kyphosis of the upper thoracic spine, which corresponds to the curvature of the posterior-convex spine of the upper thoracic spine (the upper thoracic kyphosis angle). In FIG. 2, a sharp angle formed by a line segment obtained by extending downward a line segment connecting the T3 and the T8 and the line segment connecting the T8 and the T12 is represented by Y₄. The angle Y₄ approximates the kyphosis of the middle-to-lower thoracic spine, which corresponds to the curvature of the posterior-convex spine in the middle to the lower portion of the thoracic spine (middle-to-lower thoracic kyphosis angle). In FIG. 2, a sharp angle formed by a line segment connecting the T12 and the L3 and a line segment obtained by extending upward a line segment connecting the L3 and the S is represented by Y₅. The angle Y₅ approximates the lordosis of the lumbar spine, which corresponds to the curvature of the anterior-convex spine in the lumbar spine region (lumbar lordosis angle). The lumbar lordosis angle is generally indicated by the angle formed by the upper surface of the L1 spine body and the extension line of the upper surface of the sacrum S as shown in FIG. 2, and is an index related to the lumbar alignment.

The cervical forward slope angle, the T1 slope, the upper thoracic kyphosis angle, the middle-to-lower thoracic kyphosis angle, and the lumbar lordosis angle, which are indices of the spinal alignment described above, are values originally measured by radiography. The angles Y₁ to Y₅ are estimated approximate values based on the reference points of the body surface, which are originally not known unless being subjected to radiography.

### [1-2] Description of One Embodiment

The viewing target in the present invention includes all objects as long as the distance between the object and the user can be measured, such as a display screen of a television or the like, a visual display terminal of a computer display or an operating screen of a mobile device or the like, as well as books and pictures. However, the distance between the viewing target and the user is preferably within a range of about 5 meters, which affects the inclination of the head of the user. In the following description, the viewing target is exemplified by an operating screen of an operation terminal such as a computer display.

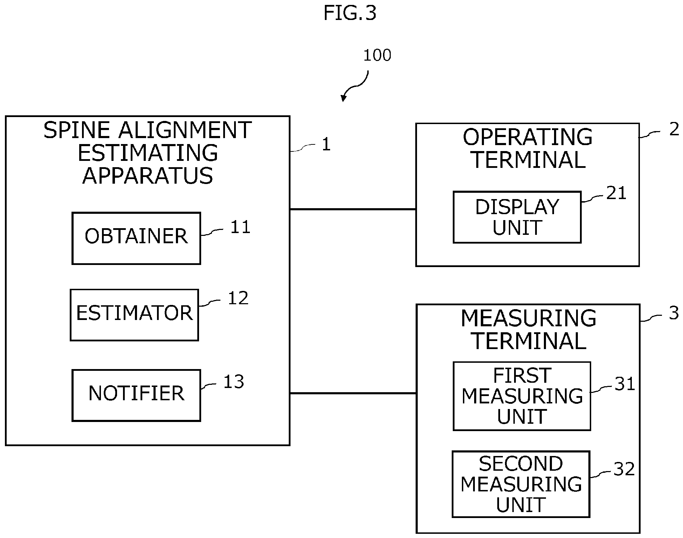

FIG. 3 is a diagram illustrating a spinal alignment estimating system 100 according to an embodiment. As shown in FIG. 3, the spinal alignment estimating system 100 may illustratively include a spinal alignment estimating apparatus 1, an operating terminal 2, and a measuring terminal 3.

The spinal alignment estimating system 100 is a system for estimating the spinal alignment of a user, and is an example of an information processing system.

The spinal alignment estimating apparatus 1, the operating terminal 2, and the measuring terminal 3 may be connected to each other via a network. FIG. 3 shows an example of wired connection, but these elements may be wirelessly connected.

The operating terminal 2 is an example of an information processing terminal operated by a user (in other words, a subject person). Examples of the operating terminal 2 include various computers such as a Personal Computer (PC) and a smart phone.

As shown in FIG. 3, the operating terminal 2 may include a display unit 21 that displays information on an operating screen (viewing target). The display unit 21 presents various kinds of information to the user through the operating screen. The presented information may include the position of the head of the user measured by a first measuring unit 31 that is to be descried below, the angle of the head of the user measured by a second measuring unit 32, and information output from a notifier 13 of the spinal alignment estimating apparatus 1 which are described below.

The measuring terminal 3 is an example of an instrument unit that measures various data in a state of being worn by a user. For example, the measuring terminal 3 may be wearable (detachable) to a user's wearing object such as eyeglasses, a headband, a necklace, an earphone, or the like worn by the user, or may be integrated (undetachable) with the wearing object of the user.

As shown in FIG. 3, the measuring terminal 3 may include the first measuring unit 31 and the second measuring unit 32. The first measuring unit 31 measures the position of the head of the user in relation to the operating screen of the operating terminal 2. In other words, the first measuring unit 31 measures the distance between the operating screen of the operating terminal 2 and the user in front of the operating screen. Alternatively, the first measuring unit 31 may measure, as the distance, a change from the initial position, which is the position of the head of the user at starting.

The second measuring unit 32 measures the angle of the head of the user. In other words, the second measuring unit 32 measures the slope angle of the head of the user facing the operating screen of the operating terminal 2. If the operating terminal 2 is a personal computer, since the angle of the operating screen is substantially constant, the angle of the head of the user can be interpreted as the angle in relation to the screen.

The measuring terminal 3 transmits the position of the head of the user measured by the first measuring unit 31 and the angle of the head of the user measured by the second measuring unit 32 to the spinal alignment estimating apparatus 1.

The spinal alignment estimating apparatus 1 is an example of a data analysis apparatus, an information processing apparatus, or a computer that analyzes data, generates display information, and manages files.

As shown in FIG. 3, the spinal alignment estimating apparatus 1 may include an obtainer 11, an estimator 12, and the notifier 13. The obtainer 11 obtains the position of the head of the user measured by the first measuring unit 31 and the angle of the head of the user measured by the second measuring unit 32 from the measuring terminal 3. The estimator 12 estimates the spinal alignment of the user based on the position and the angle of the head of the user obtained by the obtainer 11. The notifying unit 13 notifies the user of possible abnormality of the posture on the basis of the result of the estimation by the estimator 12.

### [1-3] Example of Appearance Structure of Measuring Terminal

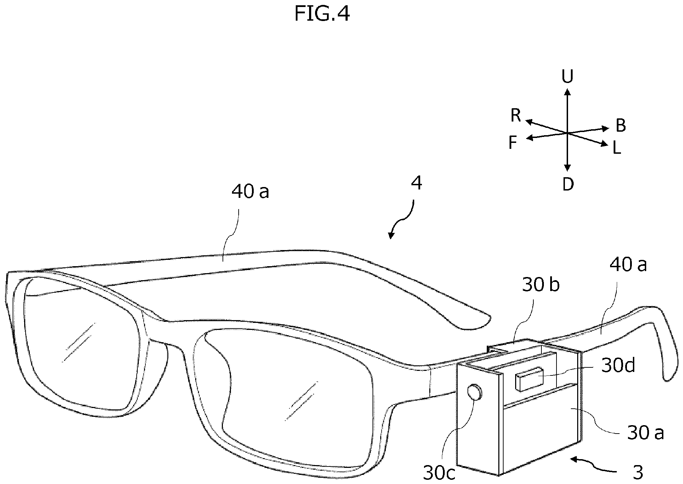

FIG. 4 is a diagram illustrating an appearance structure of the measuring terminal 3 of one embodiment. Here, the measuring terminal 3 which can be mounted on eyeglasses will be described as an example. The measuring terminal 3 is mounted on a temple 40a of eyeglasses 4 of an ordinary shape used in daily life. The measuring terminal 3 has a casing 30a, which is provided with a hook 30b for mounting on the temple 40a. Here, the casing 30a is attached to the outside (left side) of the temple 40a on the left side of the eyeglasses 4. The position of the measuring terminal 3 on the temple 40a is preferably around the user's temple, which is a position close to the eye of the user wearing the eyeglasses 4.

In the casing 30a, a position measuring device 30c and an angle measuring device 30d are accommodated. The position measuring device 30c is an example of a device that measures the position of the head of the user in relation to the operating screen of the operating terminal 2. In other words, the position measuring device 30c is an example of the first measuring unit 31. Examples of the position measuring device 30c are optical, radio, or ultrasonic distance measuring sensor or the like. Here, an example in which the camera 30c is used as the position measuring device 30c will be described.

The angle measuring device 30d is an example of a device that measures the angle of the head of the user in relation to the operating screen of the operating terminal 2. In other words, the angle measuring device 30d is an example of the second measuring unit 32. An example of the angle measuring device 30d includes an inertial sensor or a slope sensor including an acceleration sensor and a gyro. Here, the angle measuring device 30d uses an acceleration sensor 30d will be described as an example.

### [1-4] Description of Estimator

Next, description will now be made in relation to an example of a process of the estimator 12 of the spinal alignment estimating apparatus 1 described above along with an example of the spinal alignment estimating system 100 using the measuring terminal 3 that can be mounted on the eyeglasses 4 and the notebook PC 2 as the operating terminal 2.

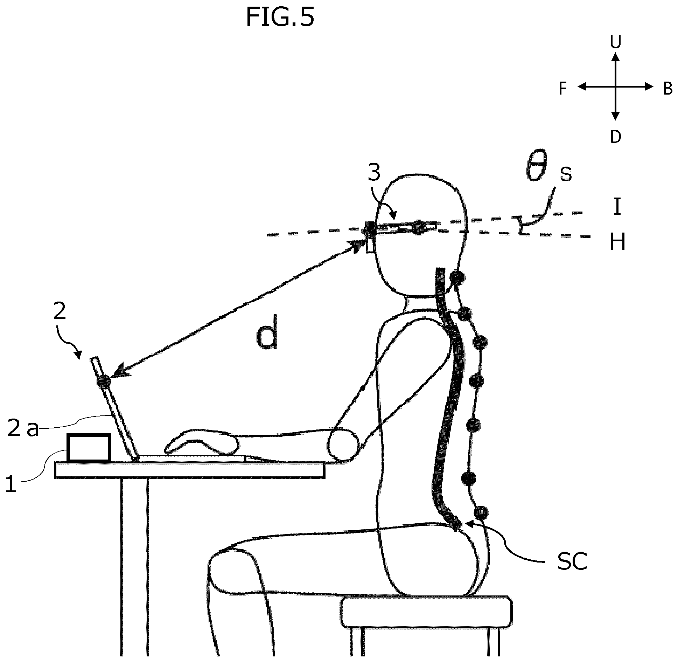

FIG. 5 is a diagram illustrating the distance between the head of the user and the VDT and the angle of the head of the user. As shown in FIG. 5, in the present embodiment as an optimum example, a case where the user wears the eyeglasses 4 attached with the measuring terminal 3 and operates the notebook PC 2 in a sitting posture will be described. The line extending along the posterior side (back surface) of the user's body indicates the spine SC. Further, black circles placed on the back surface of the user along the spine SC represent the position of the skin surface corresponding to the position of the bones related to the posture of the user in the sitting posture, and coincide with the position of the white circles in FIGS. 1 and 2.

The camera (position measuring device 30c) of the measuring terminal 3 measures the position of the head of the user in relation to the operating screen 2a, in other words, the distance d between the operating screen 2a of the notebook PC (operating terminal) 2 and the head of the user. Since the measuring terminal 3 is attached at a position near to the temple of the user, it can be said that the distance d measured by the camera 30c is the viewing distance d from the eyes of the user to the operating screen 2a.

The acceleration sensor 30d of the measuring terminal 3 measures the angle θ_{S} of the head of the user. The angle θ_{S} is the slope relative to the user's head, i.e., an inclination of the line connecting the eye and the upper aural edge with respect to the baseline (e.g., a horizontal line). When the position of the temple 40a in the front posture is indicated by a horizontal line H and the position of the temple 40a in the inclined posture is indicated by a virtual line I, it can be said that the angle θ_{S} is an angle between the horizontal line H and the virtual line I.

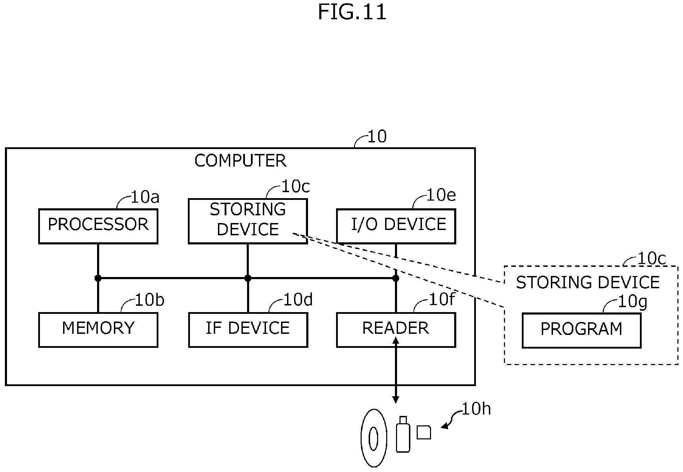

The estimator 12 estimates the spinal alignment of the user by calculating the angles formed by given parts constituting the spinal alignment using the coefficients A₁ to A₅ stored in the storing device 10c (described below with reference to FIG. 11) of the estimator 12 to be described below on the basis of the position (viewing distance d) and the angle (slope angle θ_{S}) of the head of the user obtain by the obtainer 11.

Hereafter, description will be made in relation to the correlations among the spinal alignment, the viewing distance d, and the slope angle θ_{S} obtained by experiments.

### (Experiment to examine the correlation between the measured value of the motion analyzer and the measured value of the measuring terminal 3)

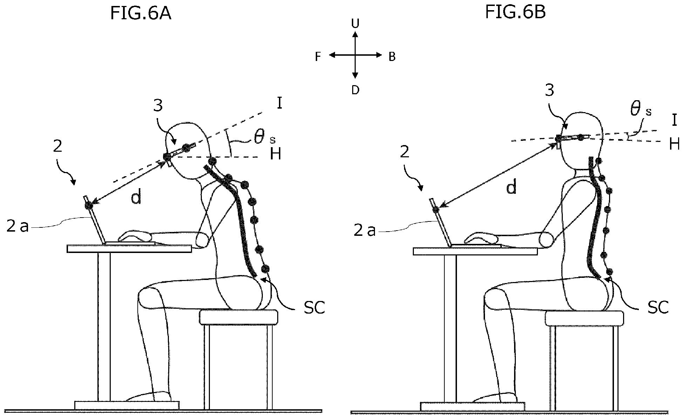

FIG. 6A and FIG.6B are diagrams illustrating fluctuation of the spinal alignment of the user in a sitting posture. An experiments was performed under predetermined conditions to examine correlations among the spinal alignment, the viewing distance d, and slope angle θ_{S}. More specifically, the spatial coordinate of the spine index was obtained with a three-dimensional motion analyzer using infrared-type high-speed cameras placed at eight locations in all directions, and simultaneously, the head slope angle θ_{S} and the viewing distance d were obtained with the measuring terminal 3. The three-dimensional motion analyzer obtained coordinate information at a spatial resolution of 1 mm and a sampling rate of 120 Hz. The measuring terminal 3 obtained the head slope angle θ_{S} and the viewing distance d at a sampling rate of 10 Hz. The experiment was performed on multiple subjects.

The subject wore the eyeglasses 4 attached with the measuring terminal 3. At the same time, markers (black circles) of a three-dimensional motion analyzer were mounted on the back surface of the subject. The markers were attached to positions corresponding to the position of the bones in relation to the posture of the subject in the sitting posture like in FIGS. 1 and 2. The markers were attached to seven points corresponding to the second cervical spine C2, the seventh cervical spine C7, the third thoracic spine T3, the eighth thoracic spine T8, the twelfth thoracic spine T12, the third lumbar spine L3, the sacrum S, additional two points front and rear sandwiching the measuring terminal 3, and one point on the operating screen 2a of the notebook PC.

The subject gradually moved away from the operating screen 2a from the initial posture (FIG. 6A) that leans forward to approach the operating screen 2a, moving the head and the neck upward and downward in harmony with a point that moves upward and downward on the operating screen until the subject comes to be in the front posture (FIG. 6B). During this period, the angles of given parts constituting the spinal alignment of the subject, the viewing distance d, and the slope angle θ_{S} were measured over time.

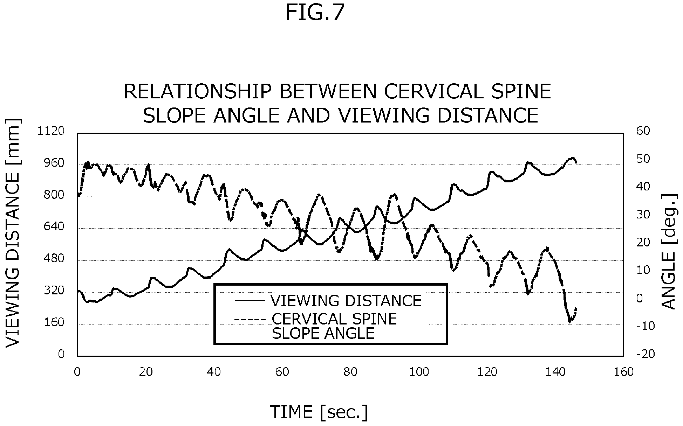

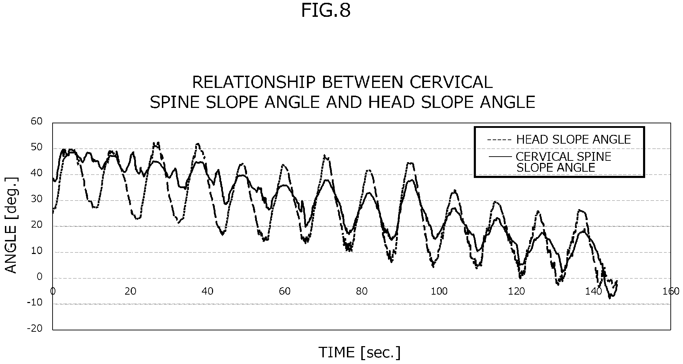

The result of the experiment showed correlations among all angles of the parts constituting the spinal alignment, the viewing distance d, and the slope angle θ_{S}. As an example, FIG. 7 shows the correlation between the cervical spine slope angle and the viewing distance, and FIG. 8 shows the correlation between the cervical spine slope angle and the head slope angle. For the C2-C7 vertical angle (cervical spine slope angle), a strong negative correlation was observed between the C2-C7 vertical angle and the viewing distance d, and a strong positive correlation was observed between the C2-C7 vertical angle and the slope angle θ_{S} (see FIGS. 7 and 8).

From the above correlations, the angles Y₁ to Y₅ (hereinafter, also referred to as an estimated pseudo-angles) formed by given parts composing the spinal alignment can be calculated by a multiple regression analysis using data obtained from the measuring terminal 3 and the regression coefficients A₁ to A₄ and intercept (coefficient) A₅ obtained using the measurement results of the actual angles Y₁ to Y₅ obtained from the three-dimensional motion analyzer. The regression analysis may be n-th order (n is a natural number), preferably in the second order or less.

Assuming that the estimated pseudo-angles Y₁ to Y₅ are targets to be estimated (object variable Yₘ) and the elements for varying the estimated pseudo-angle Y₁ to Y₅ are the slope angle θ_{S} (explanatory variable X₁) of the head and the viewing distance d (explanatory variable X₂) measured by the measuring terminal 3, these relationships are expressed by, for example, the following linear approximation expression. [Expression 1] ${Y}_{m} = {A}_{1} {X}_{1} + {A}_{2} {X}_{2} + {A}_{3} {{X}_{1}}^{2} + {A}_{4} {{X}_{2}}^{2} + {A}_{5}$

The expression 1 is a relational expression (multiple regression expression, model expression) of multiple regression analysis having multiple explanatory variables. The symbols A₁ to A₄ represent regression coefficients, and A₅ represents an intercept.

The symbol Yₘ is the angle (estimated pseudo-angle) formed by given parts constituting the spinal alignment, and m=1 to 5. Since the markers are attached to seven places corresponding to the second cervical spine C2, the seventh cervical spine C7, the third thoracic spine T3, the eighth thoracic spine T8, the twelfth thoracic spine T12, the third lumbar spine L3, and the sacrum S, the estimated pseudo-angles Y₁ to Y₅ here are pseudo values obtained by estimating the angles of the cervical forward slope angle, T1 slope, the upper thoracic kyphosis angle, the thoracic kyphosis angle, and the lumbar lordosis angle, which are significant in posture. The units for Yₘ and X₁ are [degrees] and the unit for X₂ is [mm]. Incidentally, the Yₘ to be obtained is not limited to the above angle, but may be an angle at a different location depending on the purpose, or more or less angles may be obtained.

The coefficient A₁ is the coefficient of the term of the slope angle θ_{S} of the head, and the coefficient A₃ is the coefficient of the term of the square of the slope angle θ_{S} of the head. The coefficient A₂ is the coefficient of the term of the viewing distance d, and the coefficient A₄ is the coefficient of the term of the square of the viewing distance d. The coefficient A₅ is an intercept.

The coefficients A₁ to A₅ are set differently for each user. In other words, the coefficients A₁ to A₅ are values corresponding to the attribute of a user. Examples of the attribute of the user include, for example, parameters such as age, gender, height, weight, and body shape.

The coefficients A₁ to A₄ are coefficients calculated from the correlation among the actual spinal alignment calculated by a three-dimensional motion analyzer, the slope angle θ_{S} of the head, and the viewing distance d of given subjects, measuring subjects, or users. The coefficient A₅ is the coefficient calculated in the process of calculating the coefficients A₁ to A₄. The manner of calculating the coefficient A₁ to A₅ will be described below.

Further, the coefficients A₁ to A₅ of Expression 1 are set to values corresponding to the angles formed by given parts constituting the spinal alignment. In other words, the values of the coefficients A₁ to A₅ vary with the values of the angles Y₁ to Y₅. Accordingly, the estimator 12 calculates Yₘ by substituting the viewing distance d and the slope angle θ_{S} into Expression 1 in which the coefficients A₁ to A₅ according to the attribute of the user and the Yₘ to be calculated are set.

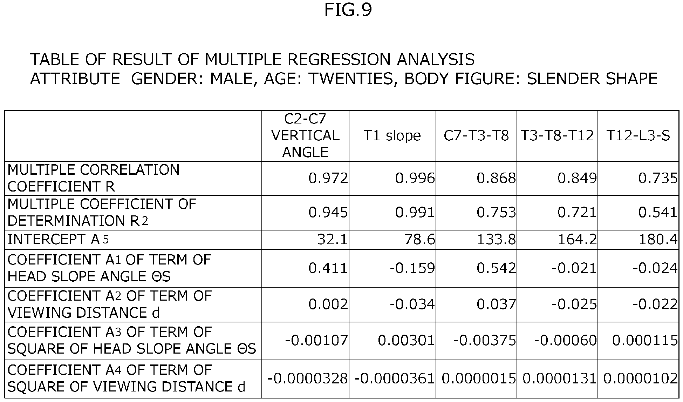

When obtaining the viewing distance d and the slope angle θ_{S}, the estimator 12 refers to the information stored in the storing device 10c and extracts the coefficients A₁ to A₅. FIG. 9 is a table showing an example of a result including the coefficients A₁ to A₅ calculated using a multiple regression analysis from values of the viewing distance d and the angle θ_{S} of the head of the user measured with the measuring terminal 3 of the present invention, and an actual measured values Y₁ to Y₅ with the three-dimensional motion analyzer. The table of the result of the multiple regression analysis is a table showing coefficients and the intercept necessary for calculating the pseudo-estimated angles.

### (Table of Results of Multiple Regression Analysis)

As shown in FIG. 9, the table of result of multiple regression analysis may include fields of, for example, "intercept A₅", "coefficient A₁ of the term of the head slope angle θ_{S}", "coefficient A₂ of the term of the viewing distance d", "coefficient A₃ of the term of the square of the head slope angle θ_{S}", and "coefficient A₄ of the term of square of viewing distance d", and optionally "multiple correlation coefficient R" and "multiple coefficient R₂ of determination" on the vertical axis. Further, the table may include the fields of "C2-C7 vertical angle", "T1 slope", "C7-T3-T8", "T3-T8-T12", and "T12-L3-S" respectively indicating Y₁ to Y₅ on the horizontal axis.

### (Calculation of the coefficients A₁ to A₅)

From the result of an experiment, it was found that all the angles of the parts constituting the spinal alignment, the viewing distance d, and the slope angle θ_{S} are expressed by the expression of the regression analysis of Expression 1. Therefore, the coefficients A₁ to A₅ were obtained by substituting, in Expression 1, the data measured by the measuring terminal 3 into X₁ and X₂ and the values measured by using a three-dimensional motion analyzer at the same time as when measured by the measuring terminal 3 into the Yₘ. The values of the coefficients A₁ to A₅ can be said to be calculated by statistically fitting the results of several measurements to an approximate expression.

In relation to regression analysis, a first-order regression analysis (first-order linear approximation) resulted in large dispersion, whereas a second-order regression analysis (second-order linear approximation) resulted in less dispersion.

As shown in the field of "multiple correlation coefficient R" in FIG. 9, the values of the correlation coefficients by the method are in the range of 0.735 to 0.972, and from this result, it can be seen that Yₘ, which are calculated by substituting the viewing distance d and the slope angle θ_{S} into X₁ and X₂ of Expression 1 in which the coefficients A₁ to A₅ set according to Yₘ, are estimated with very high accuracy.

The estimator 12 compares the calculated value of Y₁ to Y₅ with values within respective given ranges. Examples of the values of the given ranges are angles within ranges of a reference posture, that is, angles within ranges of a correct posture which is ideal from a medical point of view in which a load is not applied to the parts constituting the spinal alignment in view of the shape of the spine. As a result of the comparison, when the values of Y₁ to Y₅ are not within respective given ranges, the evaluation information indicating a bad posture is transmitted to the notifier 13. In this case, information indicating which part of the spine is particularly loaded may be transmitted.

The estimator 12 may evaluate (judge) the bad posture according to the time or frequency when the values of the Y₁ to Y₅ are not within the given ranges. For example, the estimator 12 may evaluate the bad posture based on not only the measurement time (instant) of the viewing distance d and the slope angle θ_{S} but also a value obtained by accumulating the time period or the frequency in which the values of the Y₁ to Y₅ are not in the given ranges. For example, if the time during which the values of the Y₁ to Y₅ are not within given ranges occupies 5% per hour or is counted 10 times in one day, the posture may be evaluated (determined) by comparing the ratio (value) with a given threshold. Alternatively, a value related to abnormality may be calculated through machine learning on the values of Y₁ to Y₅ when the user answers having uncomfortability, pain, or feeling abnormality in the spine as a result of responding to a questionnaire or the like, and the posture may be evaluated by using the calculated value.

The estimator 12 prepares the coefficients A₁ to A₅ obtained by using the spinal alignment estimating system 100 and the three-dimensional motion analyzer in a table form and stores the table in the storing device 10c (which will be described below with reference to FIG. 11).

This table may be an individual table unique to an individual user of the measurement subject, or may be a table generated by machine-learning on a huge amount of measured data and classifying them according to the attribute of the measurement subject. The table is selected and used by a user that is to use the spinal alignment estimating system 100 according to his/her own attribute. The attribute include gender, age, body shape, and others. In the table, the coefficients A₁ to A₅ are determined according to the combinations of gender, age, and body shape, and the user can easily estimate his/her spinal alignment with the spinal alignment estimating system 100 by selecting his/her own attribute. For example, the coefficients A₁ to A₅ of FIG. 9 correspond to "gender: male, age: twenties, body type: slender", are formed into a table to be the values of the attribute in question, and stored in the storing device 10c. Alternatively, the coefficients A₁ to A ₅ may be stored in an external server or the like.

In addition, the tables may have optimum coefficients A₁ to A₅ depending not only on the attribute but also on the state of the user. The state is a sitting posture in a chair, a sitting posture on the heels (seiza), a standing posture, a supine posture, and the like. The user can estimate the spinal alignment in any state by setting the state at the time of use in the spinal alignment estimating system 100.

### [1-5] Example of operation

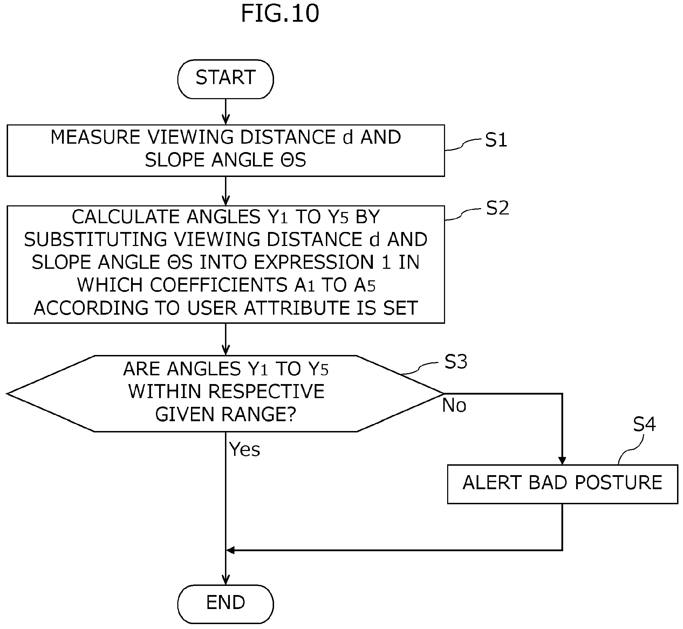

Hereinafter, an example of operating of the spinal alignment estimating system 100 will be described. FIG. 10 is a flow diagram illustrating an example of the operation of the spinal alignment estimating system 100 of the one embodiment.

The first measuring unit 31 of the measuring terminal 3 measures the viewing distance d between the operating screen 2a and the user, and the second measuring unit 32 of the measuring terminal 3 measures the slope angle θ_{S} of the head of the user facing the operating screen 2a of the operating terminal 2 (Step S1).

The obtaining unit 11 of the spinal alignment estimating apparatus 1 obtains the viewing distance d and the slope angle θ_{S} from the measuring terminal 3. The estimator 12 calculates the angles Y₁ to Y₅ of given parts of the user's spine by substituting the viewing distance d and the slope angle θ_{S} into Expression 1 in which the coefficients A₁ to A₅ corresponding to the attribute of the user are set (Step S2). Furthermore, the estimator 12, at the time of measurement, determines whether or not the values of the angles Y₁ to Y₅ in a continuous constant time (period) are within respective given ranges (Step S3).

If the values of the angles Y₁ to Y₅ are determined not to be within the given ranges (or if the time period or frequency at which the angles Y₁ to Y₅ are not within given ranges exceeds a given threshold), the notifier 13 of the spinal alignment estimating device 1 notifies the operating terminal 2 that the posture estimated from the user's spinal alignment is bad, and causes the operating terminal 2 to display an alert of the bad posture (Step S4). Then, the process ends.

On the other hand, in Step S3, if the values of the angles Y₁ to Y₅ are determined to be within the given ranges, the process ends without displaying an alert or the like in the operating terminal 2.

### [1-6] Effect

As described above, the spinal alignment estimating apparatus 1, the system for estimating a spinal alignment 100, the method for estimating a spinal alignment, the program for estimating a spinal alignment, and the computer-readable recording medium having stored therein a program for estimating a spinal alignment bring the following effects, for example.

The spinal alignment estimating apparatus 1 estimates the spinal alignment based on the viewing distance d to the user in front of the operating screen 2a of the operating terminal 2 and the slope angle θ_{S} of the head of the user facing the operating screen 2a. This makes it possible to dynamically estimate the spinal alignment over time from two pieces of information without using a large-scale device.

The spinal alignment estimating apparatus 1 estimates the spinal alignment of the user by calculating the angles Y₁ to Y₅ formed by given parts constituting the spinal alignment. This allows accurate estimation of the spinal alignment of the user in the sitting posture.

On the basis of A₁ to A₅ calculated through the experiment shown in FIG. 6A and FIG.6B, the spinal alignment estimating apparatus 1 calculates the angles Y₁ to Y₅ formed by given parts of the spinal alignment of the user. This makes it possible to obtain a value very close (highly correlated with) to the data value obtained by the three-dimensional motion analyzer. Therefore, the spinal alignment of the user can be estimated more accurately.

The spinal alignment estimating apparatus 1 notifies the user of an abnormal posture when the angle Y₁ to Y₅ formed by given parts constituting the spinal alignment calculated by the estimator 12 are not within given ranges. In response, the user can voluntarily correct his/her posture.

### [1-7] Example of Hardware Configuration of Spinal alignment Estimating Apparatus

FIG. 11 is a block diagram illustrating an example of a hardware configuration of a computer that achieves the function of the spinal alignment estimating apparatus 1. When multiple computers are used as the hardware resources that achieve the function of the spinal alignment estimating apparatus 1, each computer may have the hardware configuration illustrated in FIG. 11.

As illustrated in FIG. 11, the computer 10 may exemplarily include a processor 10a, a memory 10b, a storing device 10c, an IF (Interface) device 10d, an IO (Input/Output) device 10e, and a reader 10f as the HW configuration.

The processor 10a is an example of an arithmetic processing apparatus that performs various controls and arithmetic operations. The processor 10a may be communicably connected to the blocks in the computer 10 to each other via a bus 10i. The processor 10a may be a multiprocessor including multiple processors, a multi-core processor including multiple processor cores, or a configuration including multiple multi-core processors.

An example of the processor 10a is an Integrated Circuit (IC) such as a Central Processing Unit (CPU), a Micro Processing Unit (MPU), a Graphics Processing Unit (GPU), an Accelerated Processing Unit (APU), a Digital Signal Processor (DSP), an Application Specific IC (ASIC), and a Field-Programmable Gate Array (FPGA). Alternatively, the processor 10a may be a combination of two or more ICs exemplified as the above.

The memory 10b is an example of a HW device that stores information such as various data pieces and a program. An example of the memory 10b includes one or both of a volatile memory such as the Dynamic Random Access Memory (DRAM) and a non-volatile memory such as the Persistent Memory (PM).

The storing device 10c is an example of a HW device that stores information such as various data pieces and programs. Examples of the storing device 10c is various storing devices exemplified by a magnetic disk device such as a Hard Disk Drive (HDD), a semiconductor drive device such as an Solid State Drive (SSD), and a non-volatile memory. Examples of a non-volatile memory are a flash memory, a Storage Class Memory (SCM), and a Read Only Memory (ROM).

In addition, the storing device 10c may store a program 10g (program for estimating a spinal alignment) that implements all or part of the functions of the computer 10. For example, the processor 10a of the spinal alignment estimating apparatus 1 can implement the function of the spinal alignment estimating apparatus 1 illustrated in FIG. 3 by, for example, expanding the program 10g stored in the storing device 10c onto the memory 10b and executing the expanded program.

The IF device 10d is an example of a communication IF that controls connection to and communication with a network. For example, the IF device 10d may include an adaptor compatible with a Local Area Network (LAN) such as Ethernet (registered trademark) and an optical communication such as Fibre Channel (FC). The adaptor may be compatible with one of or both of wired and wireless communication schemes. For example, the spinal alignment estimating apparatus 1 may be communicably connected with the operating terminal 2 or the terminal 3 via the IF device 10d. Further, the program 10g (program for estimating a spinal alignment) may be downloaded from a network to a computer 10 through the communication IF and then stored into the storing device 10c, for example.

The I/O device 10e may include one of or both of an input device and an output device. Examples of the input device are a keyboard, a mouse, and a touch screen.
Examples of the output device are a monitor, a projector, a printer, and an audio device.

The reader 10f is an example of a reader that reads information of data and a program recorded on a recording medium 10h. The reader 10f may include a connecting terminal or a device to which the recording medium 10h can be connected or inserted. Examples of the reader 10f include an adapter conforming to, for example, Universal Serial Bus (USB), a drive apparatus that accesses a recording disk, and a card reader that accesses a flash memory such as an SD card. The program 10g (e.g., a program for estimating a spinal alignment) may be stored in the recording medium 10h, and the reader 10f may read the program 10g from the recording medium 10h and store the read program 10g (program for estimating a spinal alignment) into the storing device 10c.

An example of the recording medium 10h is a non-transitory computer-readable recording medium such as a magnetic/optical disk, and a flash memory. Examples of the magnetic/optical disk include a flexible disk, a Compact Disc (CD), a Digital Versatile Disc (DVD), a Blu-ray disk, and a Holographic Versatile Disc (HVD). Examples of the flash memory include a semiconductor memory such as a USB memory and an SD card.

The HW configuration of the computer 10 described above is merely illustrative. Accordingly, the computer 10 may appropriately undergo increase or decrease of HW (e.g., addition or deletion of arbitrary blocks), division, integration in an arbitrary combination, and addition or deletion of the bus. For example, at least one of the I/O device 10e and the reader 10f may be omitted in the spinal alignment estimating apparatus 1.

The operating terminal 2, which is an example of the information processing terminal, may be achieved by the same HW configuration as that of the computer 10 described above.

For example, the processor 10a of the operating terminal 2 can achieve the function as the operating terminal 2 shown in FIG. 3 by expanding a program 10g (e.g., program for estimating a spinal alignment) stored in the storing device 10c on the memory 10b and executing the expanded program 10g.

The operating terminal 2 shown in FIG. 3 may include an input device which is an example of the I/O unit 10e. Further, the processor 10a of the operating terminal 2 may transmit information (e.g., user attribute) input by the user through the input device to the spinal alignment estimating apparatus 1 via the IF device 10d.

### [2] Miscellaneous

The technique according to the above-described embodiment can be implemented after being changed or modified as follows.

In the above embodiment, the measurement of the viewing distance d is accomplished by the measuring terminal 3. Alternatively, if a camera is built in the operating terminal 2, the distance d may be measured with the camera. In addition, although the viewing distance d and the slope angle θ_{S} are measured by different apparatuses, these values may be measured using only a three-axis acceleration sensor, for example.

The spinal alignment estimating apparatus 1 may be incorporated in the operating terminal 2. In this alternative, the notification by the notifier 13 may be displayed on the display unit 21.

The spinal alignment estimating apparatus 1 may be integrated with the measuring terminal 3. The display of the abnormality of the posture to the user may be performed by any of the spinal alignment estimating apparatus 1, the operating terminal 2, and the measuring terminal 3. The alerting method may be any method as long as it is detected by a human in addition to method utilizing the visual sense and the auditory sense.

The coefficients A₁ to A₅ are only coefficients related to the slope angle θ_{S} of the head or the viewing distance , and are not limited to the values obtained in the experiment calculated by the three-dimensional motion analyzer described in the above embodiment. Alternatively, the coefficients A₁ to A₅ may be calculated by capturing multiple images of user's postures on the operating screen 2a, sending the images to a given institution or agency where a three-dimensional operation analysis carries out.

The viewing distance d, the slope angle θ_{S}, the value of the coefficients A₁ to A₅, and the values of the angles Y₁ to Y₅ may be corrected according to various conditions such as measurement conditions.

The values of the angles Y₁ to Y₅ calculated by substituting multiple patterns of the viewing distance d, the slope angle θ_{S}, and the coefficients A₁ to A₅ for each attribute of a user into Expression 1 may be stored as a data file in the storing device 10c in advance. In this case, the estimator 12 may refer to the data file stored in the storing device 10c on the basis of the obtained viewing distance d and the slope angle θ_{S} and extract the values of the angles Y₁ to Y₅ calculated by using the coefficients A₁ to A₅ corresponding to the attribute of the user.

The coefficients A₁ to A₅ may be set according to the viewing target or the relationship between the viewing target and the user. For example, if the viewing target is a book or a portable device, the relationship between the viewing target and the head of the user is determined by the position of the hand with the viewing target, so that the coefficients A₁ to A₅ may be set according to the situation. Further, in cases where the viewing target is a visual display terminal exemplified by a fixed computer display and the user is seated in front of the viewing target, the positional relationship between the user and the viewing target does not largely fluctuate, and therefore the distance between the user and the viewing target is substantially constant. On the other hand, the distance to the viewing target changes with the position where the user is sitting in some circumstances. To deal with such circumstances, the coefficients A₁ to A₅ may be set according to the status (positional relationship) between the user and the viewing target.

### [Description of Symbols]

100: spinal alignment estimating system
1: spinal alignment estimating apparatus
10: computer
10a: processor
10b: memory
10c: storing device
10d: IF device
10e: I/O device
10f: reader
10g: program
10h: storing medium
11: obtainer
12: estimator
13: notifier
2: operating terminal
2a: operating screen (viewing target))
21: display unit
3: measuring terminal
30a: casing
30b: hook
30c: position measuring device, camera
30d: angle measuring device, accelerator sensor
31: first measuring unit
32: second measuring unit
4: glasses
40a: temple
SC: spine
d: viewing distance, distance
θS: slope angle, head slop angle, angle
C2: second cervical spine
C7: seventh cervical spine
T3: third thoracic spine
T8: eighth thoracic spine
T12: twelfth thoracic spine
L3: third lumbar spine
S: sacrum

## Claims

1. A spinal alignment estimating apparatus (1) comprising:
an obtainer (11) configured to obtain a position (d) of a head of a subject person measured in relation to a viewing target, and a slope angle (θ_{S}) of the head of the subject person facing an operating screen of the viewing target (2); and
an estimator (12), **characterized in that** the estimator is configured to estimate a spinal alignment of the subject person based on the position (d) and the slope angle (θ_{S}) obtained by the obtainer by calculating one or more estimated pseudo-angles (Yₘ) formed by a given part constituting the spinal alignment, the estimated pseudo-angles (Yₘ) being angles corresponding to a cervical forward slope angle, T1 slope, an upper thoracic kyphosis angle, a thoracic kyphosis angle, and a lumbar lordosis angle.

2. The spinal alignment estimating apparatus according to claim 1, wherein the estimator calculates the pseudo-angle (Yₘ) formed by the given part by using the position of the head of the subject person, the slope angle (θ_{S}) of the head of the subject person, and a coefficient according to an attribute of the subject person.

3. The spinal alignment estimating apparatus according to claim 1 or 2, further comprising a notifier (13) configured to notify, when the pseudo-angle (Yₘ) formed by the given part calculated by the estimator is outside a given range, the subject person of abnormality of a posture.

4. A system for estimating a spinal alignment (100) comprising:
an operating terminal (2);
a measuring terminal (3); and
a spinal alignment estimating apparatus (1) according to any one of claims 1-3, wherein,
the operating terminal comprises a display unit configured to display an operating screen serving as a viewing target,
the measuring terminal comprises a first measuring unit configured to measure the position (d) of the head of the subject person measured in relation to the operating screen, and a second measuring unit configured to measure the slope angle (θ_{S}) of the head of the subject person facing an operating screen of the viewing target (2).

5. A method for estimating a spinal alignment comprising:
displaying a viewing target on a display unit;
measuring a position of a head of a subject person relative to a viewing target;
measuring a slope angle (θ_{S}) of the head of the subject person;
obtaining the position and the slope angle (θ_{S}) from a measuring terminal;
**characterized by**
estimating a spinal alignment of the subject person based on the obtained position and the obtained slope angle (θ_{S}) by calculating one or more estimated pseudo-angles (Yₘ) formed by a given part constituting the spinal alignment, the estimated pseudo-angles (Yₘ) being angles corresponding to a cervical forward slope angle, T1 slope, an upper thoracic kyphosis angle, a thoracic kyphosis angle, and a lumbar lordosis angle.

6. The method according to claim 5, wherein the method calculates the pseudo-angle formed by the given part by using the position of the head of the subject person, the slope angle (θ_{S}) of the head of the subject person, and a coefficient according to an attribute of the subject person.

7. The method according to claim 5 or 6, further comprising notifying, when the calculated pseudo-angle formed by the given part is outside a given range, the subject person of abnormality of a posture.

8. A program for estimating a spinal alignment that causes a computer to execute a process comprising:
obtaining a position (d) of a head of a subject person calculated in relation to the viewing target, and a slope angle (θ_{S}) of the head of the subject person facing an operating screen of the viewing target (2); and
**characterized by**
estimating a spinal alignment of the subject person based on the obtained position and the obtained angle by calculating one or more estimated pseudo-angles (Yₘ) formed by a given part constituting the spinal alignment, the estimated pseudo-angles (Yₘ) being angles corresponding to a cervical forward slope angle, T1 slope, an upper thoracic kyphosis angle, a thoracic kyphosis angle, and a lumbar lordosis angle.

9. The program according to claim 8, wherein the process calculates the angle formed by the given part by using the position of the head of the subject person, the slope angle (θ_{S}) of the head of the subject person, and a coefficient according to an attribute of the subject person.

10. The program according to claim 8 or 9, wherein the process further comprises notifying, when the calculated pseudo-angle (Yₘ) formed by the given part is outside a given range, the subject person of abnormality of a posture.

## Patentansprüche

1. Einrichtung (1) zur Schätzung der Wirbelsäulenausrichtung, umfassend:
eine Erfassungseinheit (11), die ausgebildet ist, um eine Position (d) eines Kopfes einer Testperson, die in Bezug auf ein Betrachtungsziel gemessen wird, und einen Neigungswinkel (θ_{S}) des Kopfes der Testperson, der einem Bedienbildschirm des Betrachtungsziels (2) zugewandt ist, zu erfassen; und
eine Schätzeinheit (12), **dadurch gekennzeichnet, dass** die Schätzeinheit ausgebildet ist, um eine Wirbelsäulenausrichtung der Testperson basierend auf der Position (d) und dem Neigungswinkel (θₛ), die durch die Erfassungseinheit erfasst werden, zu schätzen, indem ein oder mehrere geschätzte Pseudowinkel (Yₘ) berechnet werden, die von einem gegebenen, die Wirbelsäulenausrichtung bildenden Teil gebildet werden, wobei die geschätzten Pseudowinkel (Yₘ) Winkel sind, die einem zervikalen Vorwärtsneigungswinkel, T1-Neigung, einem oberen thorakalen Kyphosewinkel, einem thorakalen Kyphosewinkel und einem lumbalen Lordosewinkel entsprechen.

2. Einrichtung zur Schätzung der Wirbelsäulenausrichtung nach Anspruch 1, wobei die Schätzeinheit den von dem gegebenen Teil gebildeten Pseudowinkel (Yₘ) unter Verwendung der Position des Kopfes der Testperson, des Neigungswinkels (θ_{S}) des Kopfes der Testperson und eines Koeffizienten gemäß einem Attribut der Testperson berechnet.

3. Einrichtung zur Schätzung der Wirbelsäulenausrichtung nach Anspruch 1 oder 2, weiter umfassend eine Benachrichtigungseinheit (13), die ausgebildet ist, um, wenn der von dem gegebenen Teil gebildete, durch die Schätzeinheit berechnete Pseudowinkel (Yₘ) außerhalb eines gegebenen Bereichs liegt, die Testperson über eine Anomalie einer Körperhaltung zu benachrichtigen.

4. System zum Schätzen einer Wirbelsäulenausrichtung (100), umfassend:
ein Bedienterminal (2);
ein Messterminal (3); und
eine Einrichtung (1) zur Schätzung der Wirbelsäulenausrichtung nach einem der Ansprüche 1 bis 3, wobei,
das Bedienterminal eine Anzeigeeinheit umfasst, die ausgebildet ist, um einen Bedienbildschirm anzuzeigen, der als Betrachtungsziel dient,
wobei das Messterminal eine erste Messeinheit, die ausgebildet ist, um die Position (d) des Kopfes der Testperson zu messen, die in Bezug auf den Bedienbildschirm gemessen wird, und eine zweite Messeinheit umfasst, die ausgebildet ist, um den Neigungswinkel (θₛ) des Kopfes der Testperson zu messen, der einem Bedienbildschirm des Betrachtungsziels (2) zugewandt ist.

5. Verfahren zum Schätzen einer Wirbelsäulenausrichtung, umfassend:
Anzeigen eines Betrachtungsziels auf einer Anzeigeeinheit;
Messen einer Position eines Kopfes einer Testperson relativ zu einem Betrachtungsziel;
Messen eines Neigungswinkels (θ_{S}) des Kopfes der Testperson;
Erfassen der Position und des Neigungswinkels (θₛ) von einem Messterminal;
**gekennzeichnet durch**
Schätzen einer Wirbelsäulenausrichtung der Testperson, basierend auf der erfassten Position und dem erfassten Neigungswinkel (θₛ) durch Berechnen eines oder mehrerer geschätzter Pseudowinkel (Yₘ), die von einem gegebenen, die Wirbelsäulenausrichtung bildenden Teil gebildet werden, wobei die geschätzten Pseudowinkel (Yₘ) Winkel sind, die einem zervikalen Vorwärtsneigungswinkel, T1-Neigung, einem oberen thorakalen Kyphosewinkel, einem thorakalen Kyphosewinkel und einem lumbalen Lordosewinkel entsprechen.

6. Verfahren nach Anspruch 5, wobei das Verfahren den von dem gegebenen Teil gebildeten Pseudowinkel unter Verwendung der Position des Kopfes der Testperson, des Neigungswinkels (θ_{S}) des Kopfes der Testperson und eines Koeffizienten gemäß einem Attribut der Testperson berechnet.

7. Verfahren nach Anspruch 5 oder 6, weiter umfassend das Benachrichtigen der Testperson über eine Anomalie einer Körperhaltung, wenn der berechnete Pseudowinkel, der durch den gegebenen Teil gebildet wird, außerhalb eines gegebenen Bereichs liegt.

8. Programm zum Schätzen einer Wirbelsäulenausrichtung, das einen Computer veranlasst, einen Prozess auszuführen, umfassend:
Erfassen einer Position (d) eines Kopfes einer Testperson, die in Bezug auf das Betrachtungsziel berechnet wird, und eines Neigungswinkels (θₛ) des Kopfes der Testperson, der einem Bedienbildschirm des Betrachtungsziels (2) zugewandt ist; und
**gekennzeichnet durch**
Schätzen einer Wirbelsäulenausrichtung der Testperson basierend auf der erfassten Position und dem erfassten Winkel durch Berechnen eines oder mehrerer geschätzter Pseudowinkel (Yₘ), die durch einen gegebenen Teil gebildet werden, der die Wirbelsäulenausrichtung bildet, wobei die geschätzten Pseudowinkel (Yₘ) Winkel sind, die einem zervikalen Vorwärtsneigungswinkel, einer T1-Neigung, einem oberen thorakalen Kyphosewinkel, einem thorakalen Kyphosewinkel und einem Lendenlordosewinkel entsprechen.

9. Programm nach Anspruch 8, wobei der Prozess den durch den gegebenen Teil gebildeten Winkel unter Verwendung der Position des Kopfes der Testperson, des Neigungswinkels (θₛ) des Kopfes der Testperson und eines Koeffizienten gemäß einem Attribut der Testperson berechnet.

10. Programm nach Anspruch 8 oder 9, wobei der Prozess weiter das Benachrichtigen der Testperson über eine Anomalie einer Körperhaltung umfasst, wenn der berechnete Pseudowinkel (Yₘ), der durch den gegebenen Teil gebildet wird, außerhalb eines gegebenen Bereichs liegt.

## Revendications

1. Appareil (1) d'estimation d'alignement vertébral comprenant :
un dispositif d'obtention (11) configuré pour obtenir une position (d) de la tête d'un sujet humain mesurée par rapport à une cible de visualisation, et un angle de pente (θ_{S}) de la tête du sujet humain faisant face à un écran de commande de la cible de visualisation (2) ; et
un dispositif d'estimation (12), **caractérisé en ce que** le dispositif d'estimation est configuré pour estimer un alignement vertébral du sujet humain sur la base de la position (d) et de l'angle de pente (θₛ) obtenus par le dispositif d'obtention en calculant un ou plusieurs pseudo-angles (Yₘ) estimés formés par une partie donnée constituant l'alignement vertébral, les pseudo-angles (Yₘ) estimés étant des angles correspondant à un angle de pente cervicale vers l'avant, une pente de T1, un angle de cyphose thoracique supérieure, un angle de cyphose thoracique et un angle de lordose lombaire.

2. Appareil d'estimation d'alignement vertébral selon la revendication 1, dans lequel le dispositif d'estimation calcule le pseudo-angle (Yₘ) formé par la partie donnée en utilisant la position de la tête du sujet, l'angle de pente (θ_{S}) de la tête du sujet humain et un coefficient selon un attribut du sujet humain.

3. Appareil d'estimation d'alignement vertébral selon la revendication 1 ou la revendication 2, comprenant en outre un dispositif de notification (13) configuré pour notifier, lorsque le pseudo-angle (Yₘ) formé par la partie donnée calculé par le dispositif d'estimation est en dehors d'une plage donnée, le sujet humain d'une anomalie de posture.

4. Système (100) d'estimation d'alignement vertébral comprenant :
un terminal de commande (2) ;
un terminal de mesure (3) ; et
un appareil (1) d'estimation d'alignement vertébral selon l'une quelconque des revendications 1 à 3, dans lequel,
le terminal de commande comprend une unité d'affichage configurée pour afficher un écran de commande servant de cible de visualisation,
le terminal de mesure comprend une première unité de mesure configurée pour mesurer la position (d) de la tête du sujet humain mesurée par rapport à l'écran de commande, et une deuxième unité de mesure configurée pour mesurer l'angle de pente (θₛ) de la tête du sujet humain faisant face à un écran de commande de la cible de visualisation (2).

5. Procédé d'estimation d'alignement vertébral comprenant :
l'affichage d'une cible de visualisation sur une unité d'affichage ;
la mesure d'une position de la tête d'un sujet humain par rapport à une cible de visualisation ;
la mesure d'un angle de pente (θ_{S}) de la tête du sujet humain ;
l'obtention de la position et de l'angle de pente (θₛ) à partir d'un terminal de mesure ;
**caractérisé par**
l'estimation d'un alignement vertébral du sujet humain sur la base de la position obtenue et de l'angle de pente (θₛ) obtenu en calculant un ou plusieurs pseudo-angles (Yₘ) estimés formés par une partie donnée constituant l'alignement vertébral, les pseudo-angles (Yₘ) estimés étant des angles correspondant à un angle de pente cervicale vers l'avant, une pente de T1, un angle de cyphose thoracique supérieure, un angle de cyphose thoracique et un angle de lordose lombaire.

6. Procédé selon la revendication 5, dans lequel le procédé calcule le pseudo-angle formé par la partie donnée en utilisant la position de la tête du sujet humain, l'angle de pente (θ_{S}) de la tête du sujet humain et un coefficient selon un attribut du sujet humain.

7. Procédé selon la revendication 5 ou la revendication 6, comprenant en outre la notification, lorsque le pseudo-angle calculé formé par la partie donnée est en dehors d'une plage donnée, au sujet humain d'une anomalie de posture.

8. Programme d'estimation d'alignement vertébral qui amène un ordinateur à mettre en œuvre un processus comprenant :
l'obtention d'une position (d) de la tête d'un sujet humain calculée par rapport à la cible de visualisation, et d'un angle de pente (θₛ) de la tête du sujet humain faisant face à un écran de commande de la cible de visualisation (2) ; et
**caractérisé par**
l'estimation d'un alignement vertébral du sujet humain sur la base de la position obtenue et de l'angle obtenu en calculant un ou plusieurs pseudo-angles (Yₘ) estimés formés par une partie donnée constituant l'alignement vertébral, les pseudo-angles (Yₘ) estimés étant des angles correspondant à un angle de pente cervicale vers l'avant, une pente de T1, un angle de cyphose thoracique supérieure, un angle de cyphose thoracique et un angle de lordose lombaire.

9. Programme selon la revendication 8, dans lequel le processus calcule l'angle formé par la partie donnée en utilisant la position de la tête du sujet humain, l'angle de pente (θₛ) de la tête du sujet humain et un coefficient selon un attribut du sujet humain.

10. Programme selon la revendication 8 ou la revendication 9, dans lequel le processus comprend en outre la notification, lorsque le pseudo-angle (Yₘ) calculé formé par la partie donnée est en dehors d'une plage donnée, au sujet humain d'une anomalie de posture.
